# EUROPÄISCHE PATENTSCHRIFT

(11) **EP 2 346 517 B1**
(45) Veröffentlichungstag und Bekanntmachung des Hinweises auf die Patenterteilung: **27.01.2016**
(21) Anmeldenummer: 09756698.8
(22) Anmeldetag: 02.11.2009
(51) Int. Cl.: A61K 36/11, A61P 11/00

(54) **HYDROLYSATE AUS PFLANZENEXTRAKTEN SOWIE DIESE ENTHALTENDES ANTIBAKTERIELLES MITTEL**
PLANT EXTRACT HYDROLYSATES AND ANTIBACTERIAL PRODUCT CONTAINING THE SAME
HYDROLYSATS ISSUS D'EXTRAITS VÉGÉTAUX ET AGENT ANTIBACTÉRIEN CONTENANT CES HYDROLYSATS

(30) Priorität: 31.10.2008 DE 102008054127; 05.03.2009 DE 102009011152
(43) Veröffentlichungstag der Anmeldung: 27.07.2011
(73) Patentinhaber: BIONORICA SE, 92318 Neumarkt (DE)
(72) Erfinder: POPP, Michael, 91207 Lauf-Heuchling (DE)
(74) Vertreter: Simandi, Claus
(86) Internationale Anmeldenummer: PCT/EP2009/064477
(87) Internationale Veröffentlichungsnummer: WO 2010/049542

(56) Entgegenhaltungen:
- WO-A-99/33480
- WO-A2-02/02607
- US-A1- 2004 033 278
- KROLICKA ET AL: "Stimulation of antibacterial naphthoquinones and flavonoids accumulation in carnivorous plants grown in vitro by addition of elicitors" ENZYME AND MICROBIAL TECHNOLOGY, STONEHAM, MA, US, Bd. 42, Nr. 3, 15. Januar 2008 (2008-01-15), Seiten 216-221, XP022422532 ISSN: 0141-0229
- ANI V ET AL: "Antioxidant and antibacterial activities of polyphenolic compounds from bitter cumin (Cuminum nigrum L.)" EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, Bd. 224, Nr. 1, 9. März 2006 (2006-03-09), Seiten 109-115, XP019441218 ISSN: 1438-2385
- BECKMANN S. AND GEIGER H.: "ÜBER ZWEI kÄMPFEROLGLYKOSIDE DES SUMPFSCHACHTELHALMS (EQUISETUM PALUSTRE)" PHYTOCHEMISTRY, Bd. 2, 1963, Seiten 281-287, XP002570479
- , [Online] 6. November 2006 (2006-11-06), XP002570480 Gefunden im Internet: URL:http://www.news-ticker.ch/pm.php?news_ id=4762461&aktion=nf> [gefunden am 2010-02-25]
- JOKSIC GORDANA ET AL: "Antibacterial medicinal plants Equiseti herba and Ononidis radix modulate micronucleus formation in human lymphocytes in vitro." JOURNAL OF ENVIRONMENTAL PATHOLOGY TOXICOLOGY AND ONCOLOGY, Bd. 22, Nr. 1, 2003, Seiten 41-48, XP002570821 ISSN: 0731-8898
- SCHIER W: "Equisetum arvense as medicinal plant" ZEITSCHRIFT FUR PHYTOTHERAPIE 1985 DE, Bd. 6, Nr. 4, 1985, Seiten 126-128, XP002570822 ISSN: 0722-348X
- DO MONTE FABRICIO HOFFMANN MARTINS ET AL: "Antinociceptive and anti-inflammatory properties of the hydroalcoholic extract of stems from Equisetum arvense L. in mice" PHARMACOLOGICAL RESEARCH, ACADEMIC PRESS, LONDON, GB, Bd. 49, Nr. 3, 1. März 2004 (2004-03-01), Seiten 239-243, XP009126388 ISSN: 1043-6618 [gefunden am 2003-12-02]
- CHEN G-H ET AL: "Progress in pharmacological effects of compositions of Astragalus membranaceus" ZHONGGUO XIN YAO ZAZHI - CHINESE NEW DRUGS JOURNAL, GAI-KAN BIANJIBU, BEIJING, CN, Bd. 17, Nr. 17, 1. Januar 2008 (2008-01-01), Seiten 1482-1485, XP008126582 ISSN: 1003-3734
- MIKAMO HIROSHIGE ET AL: "Effects of crude herbal ingredients on intrauterine infection in a rat model" CURRENT THERAPEUTIC RESEARCH, Bd. 59, Nr. 2, Februar 1998 (1998-02), Seiten 122-127, XP002601354 ISSN: 0011-393X

## Beschreibung

Die Erfindung betrifft ein Hydrolysat aus wenigstens einem Extrakt aus wenigstens einem Pflanzenmaterial ausgewählt aus den jeweiligen Gattungen, insbesondere die Arten *Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamilleblüten)), *Centaurium erythraea (Tausendgüldenkraut), Levisticum officinale (Liebstöckel), Rosmarinus officinalis (Rosmarin), Angelica dahurica (Sibirische Engelwurz, PinYin Name: Baizhi), Angelica sinensis (Chinesische Angelika, PinYin Name: Danggui), Artemisia scoparia (Besen-Beifuß, Pin Yin Name: Yinchen), Astragalus membranaceus (var. Mongolicus) (Traganthwurzel, Chin.: Huang-Qi), Leonurus japonicus (Löwenohr, Chin.: T'uei), Salvia miltiorrhiza (Rotwurzel-Salbei, Chin.: Danshen), Saposhnikovia divaricata (Siler, PinYin Name: Fangfeng), Scutellaria baicalensis (Baikal-Helmkraut, Banzhilian), Siegesbeckia pubescens (Himmlisches Kraut, PinYin Name: Xixianciao), Armoracia rusticana (Meerettich), Capsicum sp. (Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfrüchte, Chin.: Chuan Lian Zi), Olea europaea (Olive), Pelargonium sp. (Pelagornien), Phytolacca americana (Kermesbeeren), Primula veris (Schlüsselblume), Salix sp. (Weide), Thymus L. (Thymian), Vitex agnus castus (Mönchspfeffer), Vitis vinifera (Edle Weinrebe)*
oder eine Mischung davon sowie ein Verfahren zur Herstellung und dessen Verwendung. Ferner betrifft die Erfindung ein daraus erhältliches antibakterielles Mittel und Arzneimittel.

Imupret® (eingetragene Marke der BIONORICA AG) ist eine bekannte Mischung aus Pflanzendrogen, dass aus *Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamilleblüten)) bereit gestellt wird. Durch eine Kombination dieser sieben Heilpflanzen erhält man eine Zusammensetzung, mit der man eine für medizinische Zwecke ausreichende Wirkung erzielen kann. Diese Zusammensetzung ist besonders geeignet für die Behandlung von Atemwegsinfektionen, insbesondere rezidivierende und chronische Atemwegsinfekte, vorzugsweise Tonsillitis. Bei der Tonsillitis oder synonym Angina, (Tonsillo)Pharyngitis, sog. Mandelentzündung besteht eine Entzündung der lymphoepithelialen Gewebe des lymphatischen Rachenrings, insbesondere der Gaumenmandeln. Man unterscheidet zwischen a.) Tonsillitis *acuta*: wird meist durch Viren (ca. 80 %; Adeno-, Parainfluenzaviren) und betahämolysierende Streptokokken der Gruppe A, seltener durch Staphylo- und Pneumokokken verursacht. Die Entzündung verläuft meist mit hohem Fieber, Halsschmerzen, kloßige Sprache, Druckschmerzhaftigkeit und Schwellung der submandibulären Lymphknoten, dabei Rötung und Schwellung der Tonsillen (Angina catarrhalis), häufig einzelne Beläge (sog. Stippchen) an den Kryptenmündungen (Angina lacunaris) oder über Lymphfollikel (Angina follicularis, Pneumokokkenangina); und b.) Tonsillitis *chronica*: wird meist durch eine Mischinfektion mit anaeroben und aeroben Erregern unter Beteiligung betahämolysierender Streptokokken der Gruppe A (durch verschiedene Serotypen eigentlich Neuinfektionen) verursacht; pathololgisch-anatomisch Retention von Zelldetritus, Kryptenabszesse, Fibrosierung und Nekrose des Parenchyms; Beteiligung des peritonsillären Gewebes. Anginen; geringe Beschwerden (sog. Halskratzen), vergrößerte submandibuläre Lymphknoten, Foetor ex ore, dabei gerötete Tonsillen mit narbiger und zerklüfteter Oberfläche, peritonsillärer Druckschmerz, bei Spateldruck auf den vorderen Gaumenbogen Entleerung von Eiter und Zelldetritus aus den Krypten.

Canephron® (eingetragene Marke der BIONORICA AG) ist eine bekannte Mischung aus drei Pflanzendrogen, nämlich *Centaurium erythraea (Tausendgüldenkraut), Levisticum officinale (Liebstöckel; Pulver aus Liebstöckelwurzel), Rosmarinus officinalis (Rosmarin; Pulver aus Rosmarinblätter)*, das aus diesen Pflanzenmaterialien bereit gestellt wird. Durch eine Kombination dieser drei Heilpflanzen erhält man eine Zusammensetzung, mit der man eine für medizinische Zwecke ausreichende Wirkung erzielen kann. Diese Zusammensetzung ist besonders geeignet für die Behandlung von Harnwegsinfektionen und wirkt antientzündlich in den Harnwegen.

Weiterhin ist Bronchipret® (eingetragene Marke der BIONORICA AG) aus Thymian (*Thymus L.)* in Kombination mit Primel (*Primula veris*) oder Efeu (*Hedera helix)* für seine antibakterielle Wirkung bekannt.

Die jeweils gemahlenen Rohdrogen sowie ethanol-wäßrige Auszüge oder daraus hergestellte Trockenauszüge (herstellbar z.B. durch Abziehen des Lösungs- /Extraktionsmittels unter vermindertem Druck) der oben genannten Pflanzen haben sich durch ihre ausschließlich auf pflanzlicher Basis beruhenden Heilkraft bewährt. Die in Bronchipret®, Canephron^{®} oder Imupret® verwendeten Heilpflanzen werden sorgfältig ausgewählt, untersucht und weiterverarbeitet. Die gleichbleibende Qualität des Arzneimittels erreicht BIONORICA durch optimal erarbeitete Anzucht-und Erntestrategien und strengste Qualitätskontrolle. Jede Einzeldroge trägt dabei einen Anteil zur einzigartigen Wirksamkeit der Zusammensetzung bei.

Angelica dahurica, Angelica sinensis, Artemisia scoparia, Astragalus membranaceus (var. mongolicus), Leonurus japonicus, Salvia miltiorrhiza, Saposhnikovia divaricata, Scutellaria baicalensis und Siegesbeckia pubescens sind bekannte Vertreter der traditionellen chinesischen Medizin (TCM) und sind für zahlreiche Indikationen beschrieben.

Weiterhin existieren Pflanzengattungen und Arten, wie Armoracia rusticana, Capsicum sp., Cistus incanus, Echinacea angustifolia, Echinacea purpurea, Galphimia glauca, Melia toosendan, Olea europaea, Pelargonium sp., Phytolacca americana, Salix sp., Vitex agnus castus und Vitis vinifera, denen jeweils in verschiedenen Indikationen eine Arzneimittelwirkung zugeschrieben werden.

Es existieren infektionsrelevante Erreger, wie beispielsweise Staphylococcus aureus, Staphylococcus epidermidis, Streptococcus pyogenes, Streptococcus pneumoniae oder Haemophilus influenzae. Unter ihnen findet sich auch ein gegen Methicillin resistenter Staphylococcus aureus- Stamm, MRSA genannt. Gegen diese Bakterien entfalten Standardantibiotika wie beta-Lactam-Antibiotika, zum Beispiel Oxacillin, Penicillin und Amoxicillin zunehmend keine Wirkung, da sich durch den allzu häufigen Einsatz von Antibiotika, bei denen die Erreger nicht vollständig abgetötet werden, Resistenzen entwickelt haben. Ein zusätzliches Risiko stellen diese Keime insbesondere auf Intensivstationen ("Point of Care" Bereichen) dar, wo sie zu Lungenentzündungen, Wundinfektionen und Blutvergiftungen oder anderen lebensbedrohlichen Infektionen führen können.

Es besteht daher ein hohes Bedürfnis neue wirksame antibakterielle Mittel zur Behandlung und Prophylaxe von Infektionskrankheiten bereitzustellen.

Es ist daher Aufgabe der vorliegenden Erfindung, ein Mittel zur Verfügung zu stellen, welches eine verbesserte antibakterielle Wirksamkeit aufweist.

Überraschender Weise zeigen jedoch Hydrolysate von Extrakten aus mindestens einer Pflanzendroge aus den jeweiligen Gattungen, insbesondere die Arten *Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamilleblüten)), *Centaurium erythraea (Tausendgüldenkraut), Levisticum officinale (Liebstöckel), Rosmarinus officinalis (Rosmarin), Angelica dahurica (Sibirische Engelwurz, PinYin Name: Baizhi), Angelica sinensis (Chinesische Angelika, PinYin Name: Danggui), Artemisia scoparia (Besen-Beifuß, Pin Yin Name: Yinchen), Astragalus membranaceus (var. Mongolicus) (Traganthwurzel, Chin.: Huang-Qi), Leonurus japonicus (Löwenohr, Chin.: T'uei* ), *Salvia miltiorrhiza (Rotwurzel-Salbei, Chin.: Danshen), Saposhnikovia divaricata (Siler, PinYin Name: Fangfeng), Scutellaria baicalensis (Baikal-Helmkraut, Banzhilian), Siegesbeckia pubescens (Himmlisches Kraut, PinYin Name: Xixianciao), Armoracia rusticana (Meerettich), Capsicum sp.(Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfrüchte, Chin.: Chuan Lian Zi), Olea europaea (Olive), Pelargonium sp. (Pelagornien), Phytolacca Americana (Kermesbeeren), Primula veris (Schlüsselblume), Salix sp. (Weide), Thymus L. (Thymian), Vitex agnus castus (Mönchspfeffer), Vitis vinifera (Edle Weinrebe)*
eine verbesserte Wirkung.

Die Erfindung betrifft insbesondere ein Hydrolysat aus wenigstens einem Extrakt, welcher hergestellt ist durch Extraktion aus getrocknetem Pflanzenmaterial aus:
a.) wenigstens einer der Pflanzen, ausgewählt aus der Gruppe bestehend aus:
   aus den jeweiligen Gattungen, insbesondere die Arten *Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamilleblüten)), *Centaurium erythraea (Tausendgüldenkraut), Levisticum officinale (Liebstöckel), Rosmarinus officinalis (Rosmarin), Angelica dahurica (Sibirische Engelwurz, PinYin Name: Baizhi), Angelica sinensis (Chinesische Angelika, PinYin Name: Danggui), Artemisia scoparia (Besen-Beifuß, Pin Yin Name: Yinchen), Astragalus membranaceus (var. Mongolicus) (Traganthwurzel, Chin.: Huang-Qi), Leonurus japonicus (Löwenohr, Chin.: T'uei )*, *Salvia miltiorrhiza (Rotwurzel-Salbei, Chin.: Danshen), Saposhnikovia divaricata (Siler, PinYin Name: Fangfeng), Scutellaria baicalensis (Baikal-Helmkraut, Banzhilian), Siegesbeckia pubescens (Himmlisches Kraut, PinYin Name: Xixianciao), Armoracia rusticana (Meerettich), Capsicum sp.(Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfrüchte, Chin.: Chuan Lian Zi), Olea europaea (Olive), Pelargonium sp. (Pelagornien), Phytolacca Americana (Kermesbeeren), Primula veris (Schlüsselblume), Salix sp. (Weide), Thymus L. (Thymian), Vitex agnus castus (Mönchspfeffer), Vitis vinifera (Edle Weinrebe) ;*
   sowie einer Mischung oder Unterkombination davon,
wobei das Hydrolysat durch hydrolytische Behandlung mit einer Mineralsäure aus dem Extrakt erhältlich ist, ggfs. Entfernen des Extraktionsmittels.

Eine bevorzugte Kombination ist *Centaurium erythraea (Tausendgüldenkraut), Levisticum officinale (Liebstöckel), Rosmarinus officinalis (Rosmarin).*

Eine weitere bevorzugte Kombination ist *Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamilleblüten)).

Eine bevorzugte Kombination ist *Thymus L. (Thymian), Primula veris (Schlüsselblume) und*/*oder Hedera helix (Efeu).*

Ferner sind bei *Echinacea purpurea herba* und *radix* bevorzugt. Bei *Primula veris* sind *flos* und *radix* bevorzugt.

Die erfindungsgemäßen Pflanzen(drogen) können wie üblich für die jeweilige Pflanzendroge aus bevorzugten Pflanzenteilen gewonnen werden, wie Blatt, Wurzel, etc.

Ein bevorzugtes Hydrolysat ist dadurch gekennzeichnet, dass die Extrakte mittels eines Extraktionsmittels aus 40 - 60 Vol.-%, insbesondere 50 Vol.-% Ethanol und 40 - 60 Vol.-%, insbesondere 50 Vol.-% Wasser aus dem Pflanzenmaterial über 24h unter Rühren und anschließendem Vakuumverdampfen des Lösungsmittels herstellbar sind.

Eine weitere bevorzugte Ausführungsform der Erfindung liegt in einem Hydrolysat welches durch hydrolytisches Behandeln der Pflanzenextrakte mit Salzsäure als Mineralsäure, insbesondere mit Salzsäure einer Konzentration von 1 M bis 10 M , vorzugsweise 6 bis 9 M, insbesondere ca. 8 M bei 80 Grad Celsius bis 100 Grad Celsius, insbesondere ca. 90°C, für 30 min bis 120 min, insbesondere 40 min bis 60 min, bevorzugt ca. 45 min, erhältlich ist. In der Endlösung beträgt die Konzentration der Salzsäure vorzugsweise 1 bis 4 M, insbesondere 1 bis 2 M, insbesondere 1,3 M.

Es ist bevorzugt, die hydrolytische Behandlung der Extrakte in Gegenwart von Ethanol, insbesondere mit Wasser verdünntem Ethanol, vorzugsweise 50 Vol.-% Ethanol, durchzuführen.

Das Versetzen der Extrakte mit der Mineralsäure kann nach Entfernen des Extraktionsmittels oder mit dem Extraktionsmittel erfolgen.

Im Rahmen dieser Erfindung bedeutet "Hydrolysat" eine aus dem Extrakt der erfindungsgemäßen Pflanze(ndroge) erhaltene wässrige Phase, in der die Hydrolyseprodukte angereichert sind. Die Hydrolyse erfolgt vorzugsweise unter Einwirkung von Säure, wie Salzsäure, Phosphorsäure, Schwefelsäure, Mineralsäure, insbesondere verdünnte Mineralsäure. Der Extrakt kann beispielsweise durch einen wässrigen / ethanolischen Auszug einer Pflanze(ndroge) erhalten werden, mittels wässriger Säure versetzt werden, zur Trockne eingedampft werden und anschließend in Wasser aufgenommen werden. Die Hydrolyse bewirkt die chemische Spaltung von Inhaltsstoffen, wobei formal ein Hydrogen und ein Hydroxid an das jeweilige Spaltprodukt addiert werden. Die Hydrolyse bewirkt im Hydrolysat eine Änderung der Stoffzusammensetzung gegenüber den bisher bekannten wässrigen / ethanolischen Extrakten. Damit die Hydrolysate der vorliegenden Erfindung physiologisch gut verträglich sind, werden die Extrakte nach dem Säurebehandlungsschritt zur Trockene eingeengt, vorzugsweise in Wasser, einem Puffer oder in verdünntem Ethanol aufgenommen und gegebenenfalls mit einer pharmazeutisch akzeptablen Base neutralisiert. Hierzu kommt als Base nicht abschließend z.B. NaOH, Na₂CO₃ oder Na₂HPO₄ in Betracht.

Es hat sich völlig überraschend herausgestellt, dass die erfindungsgemäßen Hydrolysate eine antibakterielle Wirkung aufweisen.

Die Hydrolysate der vorliegenden Erfindung weisen im Allgemeinen eine signifikante antibakterielle Wirkung auf, die in ihrer Wirkbreite mit einer Antibiotikakontrolle aus Amoxicillin und Clavulansäure (Masseverhältnis 6:1) vergleichbar ist.

Die Hydrolysate der vorliegenden Erfindung können verwendet werden zur Herstellung von Mitteln mit antibakterieller Wirkung gegen infektionsrelevante Bakterien, insbesondere grampositiven *Kokken,* insbesondere *Staphylococcus aureus, Staphylococcus epidermidis, Streptococcus pyogenes, Streptococcus pneumoniae, Streptococcus mutans und*/*oder gramnegativen Stäbchenbakterien, insbesondere Haemophilus influenzae und* / *oder MRSA (Methicillin-resistenter Staphylococcus aureus).*

Die Hydrolysate wurden im Rahmen der vorliegenden Erfindung gegen folgende HNO-und atemwegsrelevante Erreger getestet und für wirksam befunden: *Staphylococcus aureus (ATCC 25923), Staphylococcus epidermidis (ATCC 12228), Streptococcus pneumoniae (DSMZ 20566), Streptococcus pyogenes (DSMZ 20565), Streptococcus mutans (ATCC 35668), Haemophilus influenzae (DSMZ 4690), Klebsiella pneumoniae (ATCC 13883),* und *Enterococcus casseliflavus (VRE) (DSMZ 20680)* sowie gegen die Darmbakterien *Escherichia coli (ATCC 25922), Enterococcus faecalis (VRE) (ATCC 19433)* und *Pseudomonas aeruginosa.*

In vorteilhafter und an sich bekannter Weise können die Hydrolysate der vorliegenden Erfindung zur Herstellung eines antibakteriellen Mittels verwendet werden. Derartige antibakterielle Mittel können zum Schutz und/oder zur Behandlung von Infektionen vorzugsweise oral oder topisch auf Haut und Schleimhäuten, Gaumen in Form einer ihrer Anwendung entsprechenden galenischen Formulierung eingesetzt werden.

Die galenischen Formulierungen der Hydrolysate / antibakteriellen Mittel der vorliegenden Erfindung sind dadurch gekennzeichnet, dass die oralen Formulierungen wie Dragees, Tabletten, Filmtabletten, Pulver, Kapseln oder flüssige Verdünnungen, insbesondere Tropfen, Säfte oder Sirupe umfassen.

Bei Einsatz zur topischen Applikation eignen sich insbesondere Sprays, Salben, Emulsionen, Puder, Pulver, flüssige oder feste Präparate für die Inhalation, Kompressen, Wund- und Zahnfleischeinlagen, Tamponaden, Tonsillenpinsellösungen, Gurgellösungen oder Spüllösungen für Nase und Ohr. Dabei sind die oben angesprochenen Tamponaden auch für zahnärztliche Anwendungen geeignet.

Zur Verwendung der erfindungsgemäßen Hydrolysate als Spüllösungen für Nase und Ohr werden diese vorteilhaft in Kombination mit physiologischen oder hyperosmolaren Konzentrationen von Salzen oder Salzgemischen, eingesetzt. Für den Einsatz als antibakterielles Mittel hat sich herausgestellt, dass ein als Lyophilisat vorliegendes Präparat viele Vorteile hat, nämlich insbesondere bei der Lagerung und der Langzeitstabilität. Die antibakteriellen Mittel der vorliegenden Erfindung können selbstverständlich die pharmazeutisch üblichen Hilfsstoffe enthalten.

Bei mikrobiologischen Untersuchungen am Institut für Hygenie an der medizinischen Universität Innsbruck hat sich überraschenderweise herausgestellt, dass die erfindungsgemäßen Hydrolysate eine breite, teilweise ausgeprägte antibakterielle Wirkung gegen haut-, atemwegs -und HNO-relevante Erreger zeigen, die in entsprechenden Tests auf ihre antibakterielle Wirkung erheblich stärker ausgeprägt waren als dies bei unhydrolysierten Extrakten der Fall war. So ergab sich beispielsweise in antibakteriellen Empfindlichkeitstestungen mit dem Agardiffusionstest nach Mueller-Hinton (Mueller, H.J. and Hinton, J. (1941): A protein-free medium for primary isolation of the Gonococcus and Meningococcus. Proc. Soc. Expt. Biol. Med.; 48:330-333), dass von den hydrolysierten Einzeldrogenextrakten die erfindungsgemäßen Hydrolysate gegen mehrere Erreger wirksam waren und die Mehrzahl von unhydrolysierten Mischungen überraschender Weise im Agardiffusionstest praktisch keine antibakterielle Wirkung gegen das getestete Bakterienreferenzpanel zeigte.

Wesentlich ist zudem, dass diese jeweils erzielte antibakterielle Wirksamkeit einen pallativen und curativen Effekt der erfindungsgemäßen Pflanzen(drogen) verbessert, auch in einer jeweiligen Kombination.

Die Hydrolysate und das antibakterielle Mittel der vorliegenden Erfindung können somit vorteilhaft bei der Behandlung von durch Erreger ausgelösten Infektionen eingesetzt werden. Die ggfs. nur entzündungshemmende Wirkung der untersuchten Drogen und Drogenmischungen wird durch die zusätzliche antibakterielle Wirkung ergänzt, wodurch eine Infektion der Atemwege durch Abtöten der bakteriellen Erreger vollständig zum Erliegen kommt. Durch die zum Einsatz kommende medizinische Wirkung auf Basis mindestens einer ausgewählten Heilpflanze wird ein von einer Infektion betroffener Patient auf schonende Art und Weise ohne synthetisch-chemische Komponenten behandelt. Die erfindungsgemäßen Präparate zeichnen sich zudem durch eine gute Verträglichkeit im Hinblick auf seine Wechselwirkungen mit anderen Medikamenten und im Hinblick auf die selten auftretenden Nebenwirkungen aus. Das antibakterielle Mittel der vorliegenden Erfindung ist besonders gegen folgende Erreger wirksam, wobei sie speziell gegen grampositive Kokken wie Staphylococcus aureus, Staphylococcus epidermidis, Streptococcus pyogenes, Streptococcus pneumoniae, und Klebsiella gegen gramnegative Stäbchenbakterien wie Haemophilus influenzae, Pseudomonas aeruginosa sowie gegen Enterobacteriaceae faecalis (VRE), Enterobacteriaceae casseliflavus und E. coli antibakterielle Wirksamkeit zeigt. Somit betrifft die vorliegende Erfindung auch die Verwendung der erfindungsgemäßen Hydrolysate und antibakteriellen Mittel zur Herstellung eines Arzneimittels zur Behandlung von Infektionen, insbesondere durch atemwegs- und HNO-relevante Erreger ausgelöste Infektionen im Atemwegs- und HNO-Bereich.

In einer weiteren Ausführungsform betrifft die Erfindung ein Verfahren zur Herstellung von Hydrolysaten, erhältlich aus mindestens einem wässrigen/ethanolischen Extrakt aus mindestens einer erfindungsgemäßen Pflanze(ndroge), wobei vorzugsweise ein wässrig/ethanolischer Extrakt mit wässriger Säure versetzt wird und anschließend die löslichen Fraktionen gesammelt werden (= Hydrolysat).

Die Erfindung betrifft ebenfalls ein Verfahren zur Herstellung eines antibakteriellen Mittels, wobei aus mindestens einer erfindungsgemäßen Pflanze(ndroge) in einem ersten Schritt ein vorzugsweise wässrig /ethanolischer Extrakt hergestellt wird und in einem zweiten Schritt der erhaltene Extrakt mit einer wässrigen Säure versetzt wird und die wässrigen Fraktionen gesammelt und ggfs. getrocknet werden.

In einer weiteren Ausführungsform der Erfindung können die erhaltenen Hydrolysate in eine Trockenmasse überführt werden. In einer bevorzugten Ausführungsform wird eine Gefriertrocknung der Hydrolysate durchgeführt. Andere Trocknungsverfahren sind jedoch ebenfalls einsetzbar.

Sowohl die erfindungsgemäßen Hydrolysate als auch deren wässrige Suspensionen, Trockenmassen zeigen eine antibakterielle Wirkung. Daher betrifft die Erfindung ein entsprechendes antibakterielles Mittel oder die Verwendung der erfindungsgemäßen Hydrolysate als antibakterielles Mittel.

Die Erfindung betrifft ebenfalls ein Arzneimittel, bestehend aus einem erfindungsgemäßen Hydrolysat der genannten Pflanzen(drogen), wie oben beschrieben, oder Trockenmasse oder einem erfindungsgemäßen antibakteriellen Mittel zur Behandlung von Infektionen, insbesondere Atemwegsinfektionen oder die Verwendung der erfindungsgemäßen antibakteriellen Mittel als Arzneimittel.

Der Begriff "Infektion" umfasst sämtliche Erkrankungen, die durch den invasiven Einfall von Bakterien im oder am menschlichen Körper oder Säugetier eine bakterielle Infektionserkrankung verursacht. Die Entstehung einer Infektionskrankheit wird von den infektiösen (Übertragbarkeit bzw. Kontagiosität, Haftfähigkeit bzw. Tenazität, Eindringungsvermögen bzw. Invasivität, Vermehrungsvermögen bzw. Vitalität) und pathogenen Eigenschaften des Bakteriums (Pathogenität) wesentlich bestimmt. Es wird unterschieden: a) parenteral: perkutan (über die Haut), permukös (über die Schleimhäute), Inhalationsinfektion; b) enteral (über den Darm); c) über eine Wunde; d) direkt von Mensch oder Säugetier zu Mensch oder Säugetier, z. B. als Tröpfcheninfektion, Kontaktinfektion, Staubinfektion; e) indirekt über Zwischenträger oder Zwischenwirte (Vektoren); wie z.B. eine Infektkette; mit Verläufen wie a) foudroyant (schneller Beginn, schwerster Verlauf, oft tödlich); b) akut (plötzlicher Beginn, fieberhafter Verlauf über Tage); c) chronisch (allmählicher Beginn, subfebriler Verlauf über Wochen, Monate oder Jahre); d) rezidivierend (wiederholt auftretend, meist mit akut verlaufenden fieberhaften Krankheitsschüben); e) latent (klinische stumme Phasen über Monate bis Jahre). Die erfindungsgemäßen Arzneimittel sind für alle genannten Infektionstypen geeignet.

Der Begriff "Atemwegsinfektionen" umfasst sämtliche Formen der Entzündung und / oder Infektionen der Atemwege verursacht durch Bakterien, insbesondere die Tonsillitis, Sinusitis und Rhinitis. "Atemwege" bedeutet der Respirationstrakt bis hin zu den Alveolen; die oberen Atemwege umfassen die Nasenhöhle mit ihren Nebenhöhlen und den Rachen, wo sich der Luftweg mit dem Nahrungsweg kreuzt. Die unteren Atemwege beginnen mit dem Kehlkopf, es folgt die Luftröhre samt der gesamten Aufzweigung des Bronchialbaums.

Ferner betrifft die Erfindung eine pharmazeutische Formulierung enthaltend ein erfindungsgemäßes Arzneimittel bzw. antibakterielles Mittel.
Die antibakteriellen Mittel können in Form von pharmazeutischen Zubereitungen in Dosierungseinheiten zubereitet werden. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben, einem Drittel oder einem Viertel einer Tagesdosis entspricht.
Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.
Als bevorzugte pharmazeutische Formulierungen seien in flüssiger Form als saure, wässrige, wässrig/saure, wässrig/organische, wässrig/saure/organische, wässrig/alkoholische, wässrig/saure/alkoholische oder organische Formulierungen, ferner Tabletten, Dragees, Kapseln, Pillen, Granulate, Suppositorien, Lösungen, Saft, Suspensionen und Emulsionen, Pasten, Salben, Gele, Cremes, Lotions, Puder und (Nasen)Sprays genannt. Tabletten, Dragees, Kapseln, Pillen und Granulate können den oder die Wirkstoffe neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.
Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können. Der oder die Wirkstoffe können gegebenenfalls mit einem oder mehreren der oben angegebenen Trägerstoffe auch in mikroverkapselter Form vorliegen.
Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.
Salben, Pasten, Cremes und Gele können neben dem oder den Wirkstoffen die üblichen Trägerstoffe enthalten, z.B. tierische und pflanzliche Fette, Wachse, Paraffine, Stärke, Tragant- Cellulosederivate, Polyethylenglykole, Silikone, Bentonite, Kieselsäure, Talkum und Zinkoxid oder Gemische dieser Stoffe.
Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel enthalten.
Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3- Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten. Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Zur Herstellung der erfindungsgemäßen Extrakte und deren Kombinationen aus den Pflanzen wird auf die technischen Lehren, die Gegenstand von EP 1368605B1, EP 0753306B1 sind, verwiesen.

Weitere Vorteile und Merkmale der vorliegenden Erfindung ergeben sich aufgrund der Beschreibung von Ausführungsbeispielen. Die nachfolgenden Beispiele dienen zur Erläuterung der Erfindung ohne die Erfindung auf diese Beispiele zu beschränken.

### Beispiele:

### Vorbereitung der Testlösungen

Die Einzeldrogen sowie Mischextrakte mit variabler Drogenkomposition wurden in 50 % EtOH/H₂O (v/v, ca. 1 g Pflanzenmaterial auf 20 ml Lösungsmittel) für 24 h unter Rühren bei Raumtemperatur extrahiert. 1,6 ml Extrakt wurden mit 320 µl 25 % HCl (entspricht 8,1 mol/L) und 80 µl 50 % EtOH versetzt und für 45 Minuten bei 90 °C hydrolysiert. Zum Vergleich wurde in einem zweiten Schritt die Hydrolyse mit 1 ml Extrakt unter Hinzugabe von 1 ml 25 % HCl unter denselben Bedingungen durchgeführt. Nach Eindampfen der Extrakte wurde der Rückstand in 1 ml sterilem Wasser aufgenommen und auf deren antibakterielle Wirkung getestet. Screening Verfahren: Auf Müller Hinton Agar Platten bzw. Müller Hinton Agar Platten mit 5 % Hammelblut, die eine unbekannte Konzentration der zu testenden Bakterien enthielten, wurden 80 µl Testlösung aufgetragen und bei 37 °C für 24 h inkubiert.

Spiral Platter (SP): Eine Bakterienkolonie wurde in 5 ml CASO-Boullon suspendiert und bei 37 °C für 24 h inkubiert. Der Überstand wurde nach dem Zentrifugieren der Probe abgenommen, mit 0,9 % NaCl gewaschen und auf eine Konzentration von 10⁷ cfu/ml (colony forming unit per milliliter) verdünnt. Die Testlösungen wurden 1:2, 1:20 und 1: 200 verdünnt und mit der Bakteriensuspension vermischt (für Pneumococcus und H. influenzae: 1:10, für die restlichen Pathogene 1:100). Als Positivkontrolle wurde 0,9 % NaCl verwendet. Die Proben wurden mit einem Whitley Automatic Spiral Platter (WASP) nach 0, 4 und 8 Stunden plattiert und bei 37°C für 24 Stunden inkubiert.

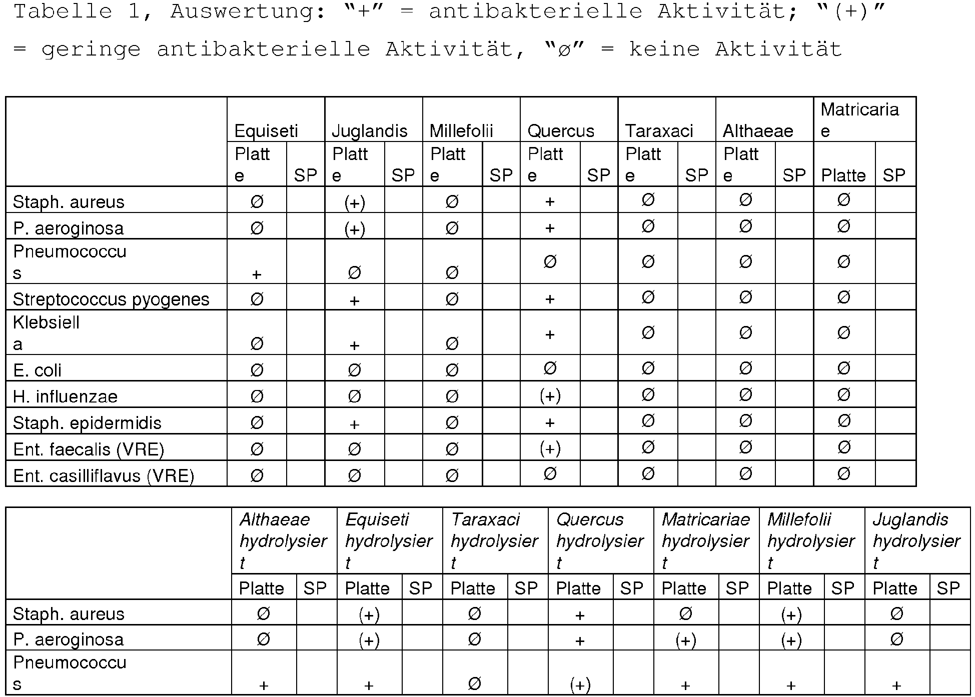

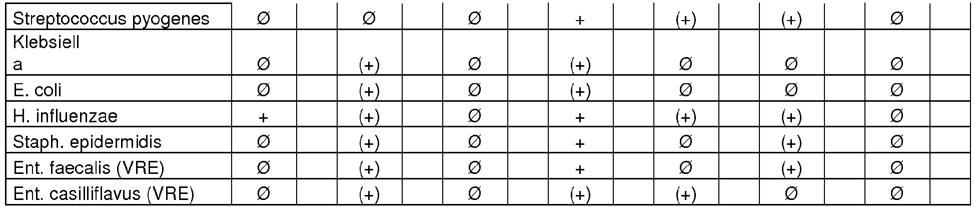

**Tabelle 2, Auswertung: "+" = antibakterielle Aktivität; "(+)" = geringe antibakterielle Aktivität, "ø" = keine Aktivität**

| Zusammensetzung Canephron: | | | | | | |
|---|---|---|---|---|---|---|
| Ergebnisse der antibakteriellen Aktivität | | | | | | |
| | *Centaurium erythraea* | | *Levisticum officinale* | | *Rosmarinus officinalis* | |
| Bacterial strains | no hydrol. | hydrol. | no hydrol. | hydrol. | no hydrol. | hydrol. |
| *Staphylococcus aureus* | Ø | + | Ø | + | + | + |
| *Pseudomonas aeruginosa* | Ø | + | Ø | + | Ø | + |
| *Streptococcus pneumonia* | Ø | + | Ø | + | (+) | + |
| *Streptococcus pyogenes* | Ø | + | Ø | + | + | + |
| *Klebsiella pneumoniae* | Ø | + | Ø | + | (+) | (+) |
| *Escherichia coli* | Ø | (+) | Ø | + | Ø | Ø |
| *Haemophilus influenzae* | Ø | (+) | Ø | + | Ø | + |
| *Staphylococcus epidermidis* | Ø | Ø | Ø | + | + | + |
| *Enterococcus faecalis (VRE)* | Ø | Ø | Ø | (+) | + | + |
| *Enterococcus casseliflavus (VRE)* | Ø | (+) | Ø | + | + | + |

**Tabelle 3, Auswertung: "+" = antibakterielle Aktivität; "(+)" = geringe antibakterielle Aktivität, "Ø" = keine Aktivität**

| Ergebnisse der antibakteriellen Aktivität | | | | | | |
|---|---|---|---|---|---|---|
| | Angelica dahurica | | Angelica sinensis | | Artemisia scoparia | |
| Bacterial strains | no hydrol. | hydrol. | no hydrol. | hydrol. | no hydrol. | hydrol. |
| *Staphylococcus aureus* | Ø | + | Ø | + | Ø | Ø |
| *Pseudomonas aeruginosa* | Ø | + | Ø | + | Ø | + |
| *Streptococcus pneumoniae* | Ø | + | + | + | Ø | (+) |
| *Streptococcus pyogenes* | Ø | + | Ø | + | Ø | (+) |
| *Klebsiella pneumoniae* | Ø | + | Ø | + | Ø | Ø |
| *Escherichia coli* | Ø | + | Ø | + | Ø | Ø |
| *Haemophilus influenzae* | Ø | + | Ø | + | Ø | + |
| *Staphylococcus epidermidis* | Ø | + | Ø | + | Ø | Ø |
| *Enterococcus faecalis (VRE)* | Ø | + | Ø | (+) | Ø | Ø |
| *Enterococcus casseliflavus (VRE)* | Ø | + | Ø | + | Ø | Ø |

| | Saposhnikovia *divaricata* | | Scutellaria baicalensis | | Siegesbeckia pubescens | |
|---|---|---|---|---|---|---|
| Bacterial strains | no hydrol. | hydrol. | no hydrol. | hydrol. | no hydrol. | hydrol. |
| *Staphylococcus aureus* | Ø | + | Ø | + | Ø | (+) |
| *Pseudomonas aeruginosa* | Ø | + | Ø | Ø | Ø | + |
| *Streptococcus pneumoniae* | Ø | + | Ø | + | Ø | + |
| *Streptococcus pyogenes* | Ø | + | Ø | (+) | Ø | + |
| *Klebsiella pneumoniae* | Ø | + | Ø | (+) | Ø | + |
| *Escherichia coli* | Ø | + | Ø | (+) | Ø | (+) |
| *Haemophilus influenzae* | Ø | + | Ø | + | Ø | (+) |
| *Staphylococcus epidermidis* | Ø | + | Ø | Ø | Ø | (+) |
| *Enterococcus faecalis (VRE)* | Ø | + | Ø | (+) | Ø | (+) |
| *Enterococcus casseliflavus (VRE)* | Ø | + | Ø | Ø | Ø | (+) |

**Tabelle 4, Auswertung: "+" = antibakterielle Aktivität; "(+)" = geringe antibakterielle Aktivität, "Ø" = keine Aktivität**

| Ergebnisse der antibakteriellen Aktivität | | | | | | |
|---|---|---|---|---|---|---|
| | Armoracia rusticana | | Capsicum sp. | | | |
| Bacterial strains | no hydrol. | hydrol. | no hydrol. | hydrat. | | |
| *Staphylococcus aureus* | Ø | + | Ø | + | | |
| *Pseudomonas aeruginosa* | Ø | + | Ø | + | | |
| *Streptococcus pneumoniae* | Ø | + | + | + | | |
| *Streptococcus pyogenes* | Ø | + | Ø | + | | |
| *Klebsiella pneumoniae* | Ø | + | Ø | + | | |
| *Escherichia coli* | Ø | + | Ø | + | | |
| *Haemophilus influenzae* | Ø | + | Ø | + | | |
| *Staphylococcus epidermidis* | Ø | + | Ø | + | | |
| *Enterococcus faecalis (VRE)* | Ø | + | Ø | + | | |
| *Enterococcus casseliflavus (VRE)* | Ø | + | Ø | + | | |

| | Cistus incanus | | Ech. purpurea radix | | Ech. purpurea herba | |
|---|---|---|---|---|---|---|
| Bacterial strains | no hydrol. | hydrol. | no hydrol. | hydrol. | no hydrol. | hydrol. |
| *Staphylococcus aureus* | + | + | Ø | + | Ø | (+) |
| *Pseudomonas aeruginosa* | Ø | + | Ø | + | Ø | + |
| *Streptococcus pneumoniae* | (+) | + | Ø | + | Ø | + |
| *Streptococcus pyogenes* | (+) | + | Ø | + | Ø | + |
| *Klebsiella pneumoniae* | Ø | + | Ø | + | Ø | + |
| *Escherichia coli* | Ø | + | Ø | + | Ø | (+) |
| *Haemophilus influenzae* | Ø | + | Ø | + | Ø | (+) |
| *Staphylococcus epidermidis* | + | + | Ø | + | Ø | Ø |
| *Enterococcus faecalis (VRE)* | + | + | Ø | + | Ø | + |
| *Enterococcus casseliflavus (VRE)* | Ø | + | Ø | + | Ø | Ø |
| Ech. = Echinacea | | | | | | |

| | Galphimia glauca | | Hedera helix | | Melia toosendan | |
|---|---|---|---|---|---|---|
| Bacterial strains | no hydrol. | hydrol. | hydrol. | hydrol. | no hydrol. | hydrol. |
| *Staphylococcus aureus* | (+) | (+) | 0 | (+) | Ø | + |
| *Pseudomonas aeruginosa* | 0 | Ø | 0 | + | Ø | + |
| *Streptococcus pneumoniae* | Ø | + | (+) | + | + | + |
| *Streptococcus pyogenes* | (+) | + | + | (+) | Ø | + |
| *Klebsiella pneumoniae* | Ø | Ø | Ø | + | Ø | + |
| *Escherichia coli* | 0 | Ø | 0 | (+) | Ø | + |
| *Haemophilus influenzae* | 0 | (+) | + | (+) | Ø | + |
| *Staphylococcus epidermidis* | (+) | (+) | (+) | Ø | Ø | + |
| *Enterococcus faecalis (VRE)* | Ø | Ø | Ø | Ø | Ø | + |
| *Enterococcus casseliflavus (VRE)* | Ø | + | Ø | + | Ø | + |

| | Olea europaea | | Pelargonium sp. | | Phytolacca americana | |
|---|---|---|---|---|---|---|
| Bacterial strains | no hydrol. | hydrol. | no hydrol. | hydrol. | no hydrol. | hydrol. |
| *Staphylococcus aureus* | + | + | + | + | Ø | + |
| *Pseudomonas aeruginosa* | Ø | + | Ø | + | Ø | + |
| *Streptococcus pneumoniae* | Ø | + | Ø | + | Ø | Ø |
| *Streptococcus pyogenes* | Ø | + | + | + | Ø | Ø |
| *Klebsiella pneumoniae* | (+) | + | Ø | + | Ø | + |
| *Escherichia coli* | Ø | + | Ø | + | Ø | + |
| *Haemophilus influenzae* | Ø | + | Ø | + | Ø | Ø |
| *Staphylococcus epidermidis* | + | + | + | + | Ø | + |
| *Enterococcus faecalis (VRE)* | Ø | + | + | + | Ø | + |
| *Enterococcus casseliflavus (VRE)* | Ø | + | + | + | Ø | Ø |

| | Primula veris radix | | Salix sp. | | Thymus L. | |
|---|---|---|---|---|---|---|
| Bacterial strains | no hydrol. | hydrol. | no hydrol. | hydrol. | no hydrol. | hydrol. |
| *Staphylococcus aureus* | + | + | + | + | + | + |
| *Pseudomonas aeruginosa* | Ø | + | + | + | Ø | + |
| *Streptococcus pneumoniae* | + | + | + | + | Ø | + |
| *Streptococcus pyogenes* | + | + | + | + | + | + |
| *Klebsiella pneumoniae* | Ø | + | + | + | Ø | + |
| *Escherichia coli* | Ø | + | + | + | Ø | + |
| *Haemophilus influenzae* | (+) | + | + | (+) | Ø | (+) |
| *Staphylococcus epidermidis* | (+) | Ø | + | + | Ø | + |
| *Enterococcus faecalis (VRE)* | (+) | (+) | + | + | Ø | + |
| *Enterococcus casseliflavus (VRE)* | Ø | (+) | + | + | Ø | + |

| | Vitex agnus castus | | Vitis vinifera | | *Ech. angustifolia* | |
|---|---|---|---|---|---|---|
| Bacterial strains | no hydrol. | hydrol. | no hydrol. | hydrol. | no hydrol. | hydrol. |
| *Staphylococcus aureus* | Ø | + | + | + | Ø | + |
| *Pseudomonas aeruginosa* | Ø | + | Ø | + | Ø | + |
| *Streptococcus pneumoniae* | Ø | + | Ø | + | Ø | + |
| *Streptococcus pyogenes* | (+) | + | Ø | + | Ø | + |
| *Klebsiella pneumoniae* | Ø | + | Ø | + | Ø | + |
| *Escherichia coli* | Ø | (+) | Ø | + | Ø | + |
| *Haemophilus influenzae* | Ø | + | Ø | + | Ø | + |
| *Staphylococcus epidermidis* | Ø | (+) | Ø | + | Ø | + |
| *Enterococcus faecalis (VRE)* | Ø | + | Ø | + | Ø | + |
| *Enterococcus casseliflavus (VRE)* | Ø | (+) | Ø | + | Ø | + |

| **Disk diffusion assay of TCM drugs** | | | | | | |
|---|---|---|---|---|---|---|
| | Huang qi | | Ch. motherwort | | Red sage | |
| Bacterial strains | no hydrol. | hydrol. | no hydrol | hydrol. | no hydrol. | hydrol. |
| Staphylococcus *aureus* | Ø | + | Ø | + | + | + |
| *Pseudomonas aeruginosa* | Ø | + | Ø | + | Ø | + |
| *Streptococcus pneumoniae* | Ø | + | (+) | + | + | + |
| *Streptococcus pyogenes* | Ø | + | + | + | (+) | + |
| *Klebsiella pneumoniae* | Ø | + | (+) | + | Ø | + |
| *Escherichia coli* | Ø | + | Ø | + | Ø | + |
| *Haemophilus influenzae* | Ø | + | Ø | + | Ø | + |
| *Staphylococcus epidermidis* | Ø | + | Ø | + | + | + |
| *Enterococcus faecalis (\*/*RE)* | Ø | + | Ø | + | + | + |
| *Enterococcus casseliflavus (VRE)* | Ø | + | Ø | + | + | + |

| | | | | | | |
|---|---|---|---|---|---|---|
| Legende: Huang qi (Astragalus membranaceus), Chinese motherwort (Leonurus japonicus), Red sage (Salvia miltiorrhiza) | | | | | | |

## Patentansprüche

1. Hydrolysat aus wenigstens einem Extrakt, welcher hergestellt ist durch Extraktion aus getrocknetem Pflanzenmaterial aus:
a.) wenigstens einer der Pflanzen, ausgewählt aus der Gruppe bestehend aus mindestens einer Gattung:
*Equiseti, Juglandis, Millefolii, Quercus, Taraxaci, Althaeae, Matricariae, Centaurium, Levisticum, Rosmarinus, Angelica(e), Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis;*
sowie einer Mischung davon; oder
b.) wenigstens einer der Pflanzen, ausgewählt aus der Gruppe bestehend aus mindestens einer Art:
*Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel), *Matricariae flos* (bzw. *Flos chamomillae* (Kamilleblüten)), *Centaurium erythraea (Tausendgüldenkraut), Levisticum officinale (Liebstöckel), Rosmarinus officinalis (Rosmarin), Angelica dahurica (Sibirische Engelwurz), Angelica sinensis (Chinesische Angelika), Artemisia scoparia* (*Besen*-*Beifuß*), *Astragalus membranaceus (var. Mongolicus) (Traganthwurzel), Leonurus japonicus (Löwenohr), Salvia miltiorrhiza (Rotwurzel-Salbei), Saposhnikovia divaricata (Siler), Scutellaria baicalensis (Baikal-Helmkraut), Siegesbeckia pubescens (Himmlisches Kraut), Armoracia rusticana (Meerettich), Capsicum sp.(Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfrüchte), Olea europaea (Olive), Pelargonium sp. (Pelagornien), Phytolacca americana* (*Kermesbeeren*), *Primula veris (Schlüsselblume), Salix sp. (Weide), Thymus L. (Thymian), Vitex agnus castus (Mönchspfeffer), vitis vinifera (Edle Weinrebe) ;* sowie einer Mischung davon; oder
c.) insbesondere eine Mischung aus:
*Centaurium erythraea (Tausendgüldenkraut), Levisticum officinale (Liebstöckel), Rosmarinus officinalis (Rosmarin);*
d.) *Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel), *Matricariae flos* (bzw. *Flos chamomillae* (Kamilleblüten));
e.) *Thymus L*. *(Thymian), Primula veris (Schlüsselblume) und*/*oder Hedera helix (Efeu);*
**dadurch gekennzeichnet, dass** das Hydrolysat durch hydrolytische Behandlung mit einer Mineralsäure aus dem Extrakt erhältlich ist, wobei die Extrakte mittels eines Extraktionsmittels aus 40 - 60 Vol. -% Ethanol und 40 - 60 Vol. -% Wasser aus dem Pflanzenmaterial über 6 bis 36 h, insbesondere 12 bis 30 h, unter Rühren und ggfs. Vakuumverdampfen des Lösungsmittels herstellbar sind.

2. Hydrolysat nach Anspruch 1, **dadurch gekennzeichnet, dass** es durch hydrolytisches Behandeln der Extrakte mit Salzsäure als Mineralsäure, insbesondere einer Konzentration von 1M bis 10M, insbesondere 6M bis 9M, bei 80 -100 Grad Celsius, für 30 bis 120 min., insbesondere 40 bis 60 min. erhältlich ist und / oder die Endlösung eine Konzentration der Salzsäure von 1 bis 4 M, insbesondere 1 bis 2 M, insbesondere 1,3 M aufweist.

3. Hydrolysat nach einem der vorhergehenden Ansprüche,
**dadurch gekennzeichnet, dass** das Hydrolysat eine antibakterielle Wirkung aufweist.

4. Hydrolysat nach einem der vorhergehenden Ansprüche,
**dadurch gekennzeichnet, dass** das Hydrolysat in einer Trockenmasse oder wässrigen Suspension vorliegt, ggfs. neutralisiert ist.

5. Hydrolysat nach einem der vorhergehenden Ansprüche,
**dadurch gekennzeichnet, dass** die antibakterielle Wirkung im Vergleich zu den nicht hydrolytisch behandelten reinen Pflanzendrogen in ihrer Wirkbreite mit einer Antibiotikakontrolle aus Amoxicillin und Clavulansäure (Masseverhältnis 6:1) vergleichbar ist.

6. Hydrolysat nach einem der vorhergehenden Ansprüche,
**dadurch gekennzeichnet, dass** sich die antibakterielle Wirkung gegen haut-, HNO-und atemwegsrelevante Bakterien, insbesondere grampositiven Kokken, insbesondere Staphylococcus aureus, Staphylococcus epidermidis, Streptococcus pyogenes, Streptococcus pneumoniae, Streptococcus mutans und/oder gramnegativen Stäbchenbakterien, insbesondere Haemophilus influenzae, richtet und / oder MRSA (Methicillin-resistenter Staphylococcus aureus).

7. Hydrolysat nach einem der vorhergehenden Ansprüche,
**dadurch gekennzeichnet, dass** es in einer galenischen Formulierung vorliegt, insbesondere eine orale Formulierung wie Dragees, Tabletten, Filmtabletten, Kapseln, flüssige Verdünnungen, insbesondere Tropfen, Säfte, Sirupe oder eine Formulierung zur topischen Anwendung wie Sprays, Salben, Emulsionen, Puder, Pulver, flüssige oder feste Präparate für die Inhalation, Kompressen, Wund-und Zahnfleischeinlagen, Tamponaden, Tonsillenpinsellösungen, Gurgellösungen oder Spüllösungen für Nase und Ohr oder Lyophilisat.

8. Antibakterielles Mittel enthaltend ein Hydrolysat nach einem der Ansprüche 1 bis 7.

9. Pharmazeutische Zusammensetzung enthaltend ein Hydrolysat nach einem der vorhergehenden Ansprüche, in Form von Pulver, Granulat, Tablette oder in flüssiger Form als saure, wässrige, wässrig/saure, wässrig/organische, wässrig/saure/organische, wässrig/alkoholische, wässrig/saure/alkoholische oder organische Formulierungen, insbesondere in einer galenischen Formulierung, insbesondere eine orale Formulierung wie Dragees, Tabletten, Filmtabletten, Kapseln, flüssige Verdünnungen, insbesondere Tropfen, Säfte, Sirupe oder eine Formulierung zur topischen Anwendung wie Sprays, Salben, Emulsionen, Puder, Pulver, flüssige oder feste Präparate für die Inhalation, Kompressen, Wund-und Zahnfleischeinlagen, Tamponaden, Tonsillenpinsellösungen, Gurgellösungen oder Spüllösungen für Nase und Ohr oder Lyophilisat, samt Zusatz und Hilfsstoffe.

10. Arzneimittel umfassend ein antibakterielles Mittel nach Anspruch 8 zur Anwendung bei Infektionen, insbesondere Atemwegsinfektionen, insbesondere Tonsillitis, Sinusitis, Rhinitis, insbesondere deren Prophylaxe und Behandlung.

## Claims

1. A hydrolysate made from at least one extract, which is produced by extraction from dried plant material of:
a.) at least one of the plants, selected from the group consisting of at least one genus:
*Equiseti, Juglandis, Millefolii, Quercus, Taraxaci, Althaeae, Matricariae, Centaurium, Levisticum, Rosmarinus, Angelica(e), Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis;*
and a mixture thereof; or
b.) at least one of the plants, selected from the group consisting of at least one species:
*Equiseti herba* (horsetail herb), *Juglandis folium* (walnut leaf), *Millefolii herba* (yarrow), *Quercus cortex* (oak bark), *Taraxaci herba* (dandelion root herb), *Althaeae radix* (marshmallow root), *Matricariae flos* (or *Flos chamomillae* (chamomile flower)), *Centaurium erythraea (centaury herb), Levisticum officinale (lovage), Rosmarinus officinalis (rosemary), Angelica dahurica (dahurian angelica), Angelica sinensis (Chinese angelica), Artemisia scoparia (redstern wormwood), Astragalus membranaceus (var. Mongolicus) (Chinese milk vetch), Leonurus japonicus (lion's tail), Salvia miltiorrhiza (red sage), Saposhnikovia divaricata (siler), Scutellaria baicalensis (Baikal skullcap), Siegesbeckia pubescens (Siegesbeckia), Armoracia rusticana (horseradish), Capsicum sp. (pepper), Cistus incanus (hairy rockrose), Echinacea angustifolia (echinacea), Echinacea purpurea (echinacea), Galphimia glauca, Hedera helix (ivy), Melia toosendan (chinaberry fruit), Olea europaea (olive), Pelargonium sp. (geranium), Phytolacca americana (pokeberry), Primula veris (cowslip primrose), Salix sp. (willow), Thymus L. (thyme), Vitex agnus castus (monk's pepper), Vitis vinifera (wine grape);*
and a mixture thereof; or
c.) in particular a mixture of:
*Centaurium erythraea (centaury herb), Levisticum officinale (lovage), Rosmarinus officinalis (rosemary);*
d.) *Equiseti herba* (Equisetum stem), *Juglandis folium* (walnut leaf), *Millefolii herba* (yarrow herb), *Quercus cortex* (oak bark), *Taraxaci herba* (dandelion root herb), *Althaeae radix* (althea root), *Matricariae flos* (or *Flos chamomillae* (chamomile flower));
e.) *Thymus L. (thyme), Primula veris (cowslip primrose) and*/*or Hedera helix (ivy);*
**characterized in that** the hydrolysate can be obtained from the extract by hydrolytic treatment with a mineral acid, wherein the extracts can be produced from the plant material using an extraction agent made of 40 - 60% by volume ethanol and 40 - 60% by volume water over 6 hours to 36 hours, in particular 12 hours to 30 hours, while stirring and, optionally, vacuum evaporation of the solvent.

2. The hydrolysate according to claim 1, **characterized in that** the hydrolysate can be obtained by hydrolytic treatment of the extracts with hydrochloric acid as the mineral acid, in particular having a concentration of 1 M to 10 M, in particular 6 M to 9 M, at 80°C to 100°C, for 30 minutes to 120 minutes, in particular 40 minutes to 60 minutes and/or the final solution has a concentration of hydrochloric acid of 1 M to 4 M, in particular 1 M to 2 M, in particular 1.3 M.

3. The hydrolysate according to any one of the preceding claims, **characterized in that** the hydrolysate exhibits an antibacterial effect.

4. The hydrolysate according to any one of the preceding claims, **characterized in that** the hydrolysate is present as a dry mass or an aqueous suspension and is optionally present in neutralized form.

5. The hydrolysate according to any one of the preceding claims, **characterized in that**, as compared to the non-hydrolytically treated, pure vegetable drugs, the scope of effectiveness of the antibacterial effect is comparable to an antibiotic control made of amoxicillin and clavulanic acid (mass ratio 6:1).

6. The hydrolysate according to any one of the preceding claims, **characterized in that** the antibacterial effect is directed against skin, ENT and respiratory tract relevant bacteria, in particular gram-positive cocci, in particular Staphylococcus aureus, Staphylococcus epidermidis, Streptococcus pyogenes, Streptococcus pneumoniae, Streptococcus mutans and/or gram-negative rod bacteria, in particular Haemophilus influenzae, and/or MRSA (methicillin-resistant Staphylococcus aureus).

7. The hydrolysate according to any one of the preceding claims, **characterized in that** it is present in a galenic formulation, in particular in an oral formulation such as sugar-coated tablets, tablets, film-coated tablets, capsules, liquid dilutions, in particular drops, oral solutions, syrups, or a formulation for topical application such as sprays, ointments, emulsions, powders, liquid or solid preparations for inhalation, compresses, wound and gum dressings, tamponades, tonsil brush solutions, gargling solutions, or rinsing solutions for the nose and ears, or is present as a lyophilisate.

8. Antibacterial means containing a hydrolysate according to any one of claims 1 to 7.

9. A pharmaceutical composition containing a hydrolysate according to any one of the preceding claims, in the form of powder, granulate, tablets or in liquid form as acid, aqueous, aqueous/acidic, aqueous/organic, aqueous/acidic/organic, aqueous/alcoholic, aqueous/acidic/alcoholic or organic formulations, in particular in a galenic formulation, in particular an oral formulation such as sugar-coated tablets, tablets, film-coated tablets, capsules, liquid dilutions, in particular drops, oral solutions, syrups, or a formulation for topical application such as sprays, ointments, emulsions, powders, liquid or solid preparations for inhalation, compresses, wound and gum dressings, tamponades, tonsil brush solutions, gargling solutions, or rinsing solutions for the nose and ears, or is present as a lyophilisate, including additives and auxiliary agents.

10. A pharmaceutical comprising an antibacterial means according to claim 8 for use in the case of infections, in particular respiratory tract infections, in particular tonsillitis, sinusitis, rhinitis, in particular for the prevention and treatment thereof.

## Revendications

1. Hydrolysat constitué d'au moins un extrait, lequel est fabriqué par une extraction en partant d'une plante séchée parmi :
a.) au moins une des plantes choisie dans le groupe constitué d'au moins une espèce :
*Equiseti, Juglandis, Millefolii, Quercus, Taraxaci, Althaeae, Matricariae, Centaurium, Levisticum, Rosmarinus, Angelica(e), Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis;*
ainsi que d'un mélange de celles-ci ; ou
b.) au moins une des plantes choisie dans le groupe constitué d'au moins un type de :
*Equiseti herba* (prêle), *Juglandis folium* (feuilles de noyer), *Millefolii herba* (achillée millefeuille), *Quercus cortex* (écorce de chêne), *Taraxaci herba* (pissenlit), *Althaeae radix* (racine de guimauve), *Matricariae flos* (respectivement *Flos chamomillae* (fleurs de camomille)), *Centaurium erythraea (centaurée), Levisticum officinale (livèche), Rosmarinus officinalis (romarin), Angelica dahurica (angélique de Sibérie), Angelica sinensis (Angélique de Chine), Artemisia scoparia (armoise à balais), Astragalus membranaceus (var. Mongolicus) (racine d'astragale), Leonurus japonicus (oreille de lion), Salvia miltiorrhiza (sauge rouge), Saposhnikovia divaricata (sermontain), Scutellaria baicalensis (scutellaire latériflore), Siegesbeckia pubescens (herbe du ciel), Armoracia rusticana (raifort), Capsicum sp. (poivron), Cistus incanus (ciste), Echinacea angustifolia (échinacée), Echinacea purpurea (échinacée pourpre), Galphimia glauca, Hedera helix (lierre), Melia toosendan (baies de sureau chinois*)*, Olea europaea (olive), Pelargonium sp. (pélargoniums), Phytolacca americana (raisin d'Amérique), Primula veris (primevère), Salix sp. (saule), Thymus L. (thym), Vitex agnus castus (gatillier), Vitis vinifera (vigne cultivée) ;*
ainsi qu'un mélange de ceux-ci ; ou
c.) en particulier, un mélange de :
*Centaurium erythraea (centaurée), Levisticum officinale (livèche), Rosmarinus officinales (romarin) ;*
d.) *Equiseti herba* (prêle), *Juglandis folium* (feuilles de noyer), *Millefolii herba* (achillée millefeuille), *Quercus cortex* (écorce de chêne), *Taraxaci herba* (pissenlit), *Althaeae radix* (racine de guimauve), *Matricariae flos* (respectivement *Flos chamomillae* (fleurs de camomille)) ;
e.) *Thymus L. (thym), Primula veris (primevère) et*/*ou Hedera helix (lierre) ;*
**caractérisé en ce que** l'hydrolysat peut être obtenu à partir de l'extrait par un traitement par hydrolyse avec un acide minéral, où les extraits peuvent être fabriqués à partir des plantes au moyen d'un produit d'extraction constitué de 40 à 60 % en volume d'éthanol et de 40 à 60 % d'eau pendant 6 à 36 heures, notamment pendant 12 à 30 heures, sous agitation et éventuellement par évaporation du solvant sous vide.

2. Hydrolysat selon la revendication 1, **caractérisé en ce qu'**il peut être obtenu par un traitement des extraits par hydrolyse avec de l'acide chlorhydrique en tant qu'acide minéral, notamment à une concentration de 1 M à 10 M, en particulier, de 6 M à 9 M, entre 80 et 100 degrés Celsius, pendant 30 à 120 minutes, notamment pendant 40 à 60 minutes et/ou la solution finale présente une concentration d'acide chlorhydrique de 1 à 4 M, notamment de 1 à 2 M, en particulier de 1,3 M.

3. Hydrolysat selon l'une des revendications précédentes, **caractérisé en ce que** l'hydrolysat présente un effet antibactérien.

4. Hydrolysat selon l'une des revendications précédentes, **caractérisé en ce que** l'hydrolysat est présent sous la forme d'une matière sèche ou d'une suspension aqueuse, est éventuellement neutralisé.

5. Hydrolysat selon l'une des revendications précédentes, **caractérisé en ce que** l'effet antibactérien, par rapport à des médicaments à base de plantes pures non traitées par hydrolyse, est comparable dans son spectre d'activité avec celle d'un étalon d'antibiotiques constitué d'amoxicilline et d'acide clavulanique (rapport en masses 6 : 1).

6. Hydrolysat selon l'une des revendications précédentes, **caractérisé en ce que** l'effet antibactérien s'exerce contre des bactéries de la peau, relatives à la sphère ORL et aux voies respiratoires, notamment des coques gram positif, notamment le Staphylococcus aureus, le Staphylococcus epidermidis, le Streptococcus pyogenes, le Streptococcus pneumoniae, le Streptococcus mutans et/ou des bactéries en bâtonnets gram négatif, notamment l'Haemophilus influenzae, et/ou le SARM (Staphylococcus aureus résistant à la méthicilline).

7. Hydrolysat selon l'une des revendications précédentes, **caractérisé en ce qu'**il est présent dans une formulation galénique, notamment une formulation orale comme des comprimés enrobés, des comprimés, des comprimés pelliculés, des gélules, des solutions liquides, notamment des gouttes, des jus, des sirops, ou une formulation pour un emploi topique comme des sprays, des pommades, des émulsions, des poudres, de la poudre, des préparations liquides ou solides pour l'inhalation, des compresses, des pansements pour des plaies et les gencives, des tamponnades, des solutions de badigeonnage pour les amygdales, des solutions pour gargarismes ou des solutions de nettoyage du nez et des oreilles, ou en lyophilisat.

8. Produit antibactérien contenant un hydrolysat selon l'une des revendications 1 à 7.

9. Composition pharmaceutique contenant un hydrolysat selon l'une des revendications précédentes, sous forme de poudre, de granulés, de comprimés ou sous forme liquide comme des formulations acides, aqueuses, aqueuses/acides, aqueuses/organiques, aqueuses/acides/organiques, aqueuses/alcooliques, aqueuses/acides/alcooliques ou organiques, notamment dans une formulation galénique, notamment une formulation orale comme des comprimés enrobés, des comprimés, des comprimés pelliculés, des gélules, des solutions liquides, notamment de gouttes, de jus, de sirops ou une formulation pour un emploi topique comme des sprays, des pommades, des émulsions, des poudres, de la poudre, des préparations liquides ou solides pour l'inhalation, des compresses, des pansements pour les plaies et les gencives, des tamponnades, des solutions de badigeonnage des amygdales, des solutions de gargarisation ou des solutions de lavage pour le nez et les oreilles ou de lyophilisat, y compris des additifs et des produits auxiliaires.

10. Produit pharmaceutique comprenant un produit antibactérien selon la revendication 8 pour un emploi dans des infections, notamment des infections des voies respiratoires, notamment une amygdalite, une sinusite, une rhinite, en particulier, leur prophylaxie et leur traitement.
